# EUROPÄISCHE PATENTSCHRIFT

(11) **EP 0 749 327 B1**
(45) Veröffentlichungstag und Bekanntmachung des Hinweises auf die Patenterteilung: **24.10.2001**
(21) Anmeldenummer: 95912242.5
(22) Anmeldetag: 10.03.1995
(51) Int. Cl.: A61M 1/00, A61M 27/00

(54) **VORRICHTUNG FÜR DEN VAKUUM-WUNDVERSCHLUSS UND/ODER ZUM ABSAUGEN VON SEKRET ODER DGL.**
DEVICE FOR CLOSING WOUNDS BY VACUUM AND/OR FOR EXTRACTING SECRETION OR THE LIKE
DISPOSITIF PERMETTANT L'OCCLUSION DE PLAIES A L'AIDE DU VIDE ET/OU LE POMPAGE DE SECRETIONS OU SIMILAIRES

(30) Priorität: 10.03.1994 DE 9404048 U
(43) Veröffentlichungstag der Anmeldung: 27.12.1996
(73) Patentinhaber: Biovac Medizintechnik GmbH, 1230 Wien (AT)
(72) Erfinder: KILIAN, Frank, D-85560 Ebersberg (DE); O'HALLORAN, Michael, A-1100 Wien (AT); PIRKER, Oswald, A-1230 Wien (AT)
(74) Vertreter: Melzer, Wolfgang, Dipl.-Ing.
(86) Internationale Anmeldenummer: EP9500903
(87) Internationale Veröffentlichungsnummer: WO9524230

(56) Entgegenhaltungen:
- EP-A- 0 040 427
- EP-A- 0 082 510
- EP-A- 0 573 117
- FR-A- 2 281 769
- US-A- 4 004 590
- US-A- 4 335 723
- US-A- 4 772 256

## Beschreibung

Die vorliegende Erfindung betrifft eine Vorrichtung für den Vakuum-Wundverschluß und/oder zum Absaugen von Sekret oder dgl., die insbesondere zum Einsatz in opterativen Bereichen in Krankenhäusern und im Sanitätswesen geeignet ist.

Absaugvorrichtungen für Sekret, Blut oder dgl. und Vorrichtung als Wundverschluß nach Redon sind in vielen Bereichen des Sanitäts- und Gesundheitswesens im Einsatz. Allen bisherigen Systemen liegt dabei das Prinzip zugrunde, daß mittels eines Vakuums ein Unterdruck in einem Behälter, meistens einer, vorzugsweise durchsichtigen, Glas- oder Kunststoffflasche, erzeugt wird. Nach Redon wird mit einer in das Wundgebiet gelegten Drainage im Operationsfeld ein Unterdruck erzeugt, der die Wundflächen aneinanderpreßt, wodurch Hohlräume - z. B. bei Ausräumungen - vermieden werden. Damit ist gleichzeitig das Abtransportieren (Absaugen) von Sekret verbunden. Beim Absaugen wird durch eine Drainage im Operationsgebiet Sekret, z.B. aus einer frisch operierten Wunde, mittels des Unterdruckes in den Behälter abgesaugt. Permanentes Absaugen beschleunigt den Heilungsprozeß. Beim intraoperativen Einsatz von Absaugvorrichtungen wird Blut, das nach einer späteren Aufbereitung zur Eigentransfusion verwendet werden soll, in den Behälter abgesaugt. Somit kann dem Patienten sein eigenes Blut während oder nach einer Operation zurückgeführt werden, was die Gefahr einer Krankheitserreger-Übertragung, wie beispielsweise Hepatitis-Viren, HIV etc., beseitigt. Der Nachteil dabei ist, daß die Schläuche und der ganze Behälter zur Aufnahme des abgesaugten Sekrets oder Bluts jeweils nach Gebrauch gereinigt und neu sterilisiert oder vollständig als Einweg-Produkt entsorgt werden müssen.

Um diesen großen Aufwand zu vermeiden, ist in der DE-OS-25 36 746 die Verwendung von Einweg-Beuteln, z.B. aus Kunststoff, die in Behälter eingesetzt werden, vorgeschlagen worden. Nach dem Einsetzen des Beutels in den Behälter wird dieser durch einen Deckel verschlossen. Ein mit dem Beutel meist einstückig verbundener Schlauch führt durch eine Durchführung im Deckel zur Drainage, d. h. zum Operationsfeld. Eine weitere Öffnung im Boden des Behälters ist ständig mit einer Vakuumpumpe verbunden. Diese Vakuumpumpe erzeugt im Behälter durch das kontinuierliche Abpumpen von Luft einen ständigen Unterdruck. Für den Fall des Ausfalls der Vakuumpumpe ist das umschaltbare oder automatische Einspringen einer zentralen Sauganlage des Krankenhauses vorgesehen. Dieser Unterdruck bewirkt, daß sich der Beutel im Behälter gewissermaßen aufbläht. Dieses Aufblähen des Beutels hat nun wegen der Volumenvergrößerung des Beutels wiederum einen Unterdruck im Inneren des Beutels zur Folge, so daß das Sekret durch den Schlauch in den Beutel abgesaugt wird. Wenn der Beutel voll ist, werden einfach der benutzte Schlauch und der volle Beutel entfernt und entsorgt, und werden ein neuer, steriler Beutel und ein neuer, steriler Schlauch in den Behälter eingesetzt. Dadurch wird erstens schon bei der Lagerung viel Platz gespart, da die Beutel weniger Platz einnehmen, als die stabilen und nicht zusammenlegbaren Behälter. Zweitens entfällt die Notwendigkeit, die Behälter ständig reinigen und sterilisieren zu müssen.

Der Nachteil bei diesem Stand der Technik ist, daß die den Unterdruck im Behälter erzeugende Vakuumpumpe ständig in Betrieb sein muß, da sonst der Unterdruck im Behälter und damit die Saugwirkung im Beutel nicht aufrechterhalten werden kann. Die Notwendigkeit, diese Absaugvorrichtung ständig an eine kontinuierlich betriebene Vakuumpumpe anzuschließen, hat zahlreiche Nachteile. So ist z.B. für jede Absaugvorrichtung eine Vakuumpumpe oder ein Anschluß an eine zentrale Sauganlage erforderlich. Alternativ dazu sind bereits starre beim Hersteller vorevakuierte Behälter als Einwegbehälter aus Glas oder Kunststoff vorgeschlagen worden, insbesondere für die Redon-Anwendung. Die Lagerhaltung, aber auch die Entsorgung, sind hier äußerst problematisch und kostspielig. Herkömmliche Vorrichtungen als Vakuum-Wundverschluß und/oder zum Absaugen von Sekret sind somit für den ambulanten Einsatz nicht oder nur sehr beschränkt verwendbar.

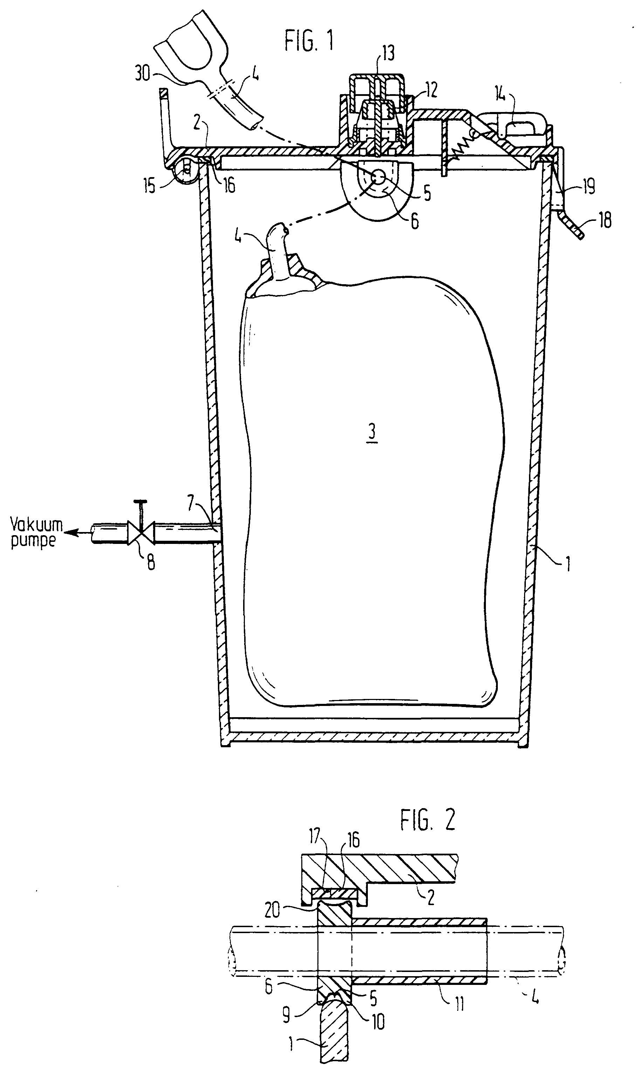

Aus der EP 0 082 510 A1 ist eine Saugflasche für medizinische Zwecke mit evakuierbarer Innenkammer und Anschlußstutzen für einen Drainageschlauch bekannt. Der Schlauch-Anschlußstutzen mündet in einem in der Innenkammer angeordneten, vom Unterdruck volumenvergrößerbaren Wegwerfbeutel, dessen Kragen am Öffnungsrand der Saugflasche vakuumdicht verspannt ist. Diese bekannte Saugflasche weist indessen den Nachteil auf, daß der Deckel entweder (Fig. 1) fest mit dem Schlauch verbunden ist und somit zusammen mit diesem sterilisiert werden muß, oder die Innenseite des Deckels (Fig. 8) durch in den Wegwerfbeutel gesaugte Flüssigkeit kontaminiert wird und somit nach jeder Benutzung gereinigt werden muß. Der Deckel gemäß dieser bekannten Saugflasche ist somit nicht dem Gehäuse zugeordnet weiterverwendbar.

Es besteht daher das dringende Bedürfnis nach einer universell einsetzbaren, leicht handhabbaren und für den ambulanten Gebrauch geeigneten, d.h. ortsunabhängigen, Vorrichtung zum Erzeugen eines Unterdrucks im Wundgebiet.

Diese Aufgabe wird durch die Merkmale des Anspruches 1 der vorliegenden Erfindung gelöst.

Dabei liegt der Hauptgedanke der vorliegenden Erfindung darin, den Behälter so verschließbar zu gestalten, daß ein einmaliges Abpumpen des Behälterinneren ausreicht, eine ausreichend lange anhaltende Saugwirkung im Beutel zu erzeugen. Das bedeutet auf der einen Seite, daß der Deckel den Behälter vakuumdicht verschließen muß. Andererseits muß auch die Öffnung, durch die mittels der Vakuumpumpe die Luft aus dem Behälterinneren abgepumpt wird, vakuumdicht verschließbar sein, z.B. durch ein Ventil. Weiterhin muß, und das ist einer der wichtigsten Punkte der Erfindung, die Durchführung des Schlauches zum Absaugen des Sekrets so ausgestaltet sein, daß sie in den Behälter vakuumdicht einsetzbar ist. Das wird durch die besondere Ausgestaltung eines am Schlauch befestigten Dichtungselementes gewährleistet, das leicht und problemlos in die dafür vorgesehene Öffnung eingesteckt werden kann und mittels dem der Schlauch durch den Deckel bzw. die Behälterwand geführt werden kann. Dadurch ist es möglich, durch einmaliges Abpumpen eine ausreichend hohe und ausreichend lang anhaltende Saugwirkung zu erzeugen. Der große Vorteil ist dabei, daß die Vakuumpumpe nach dem Abpumpen eines Behälters abgetrennt werden und zum Abpumpen weiterer Behälter verwendet werden kann. Es ist also nicht nötig, für jede Absaugvorrichtung eine Vakuumpumpe bereitzustellen. Die Sekret-Absaugvorrichtung gemäß der vorliegenden Erfindung ist somit universell und auch ambulant einsetzbar.

Hinsichtlich insbesondere der Absaugung von Blut während einer Operation zwecks späterer Wiederverwendungen kann der Beutel eine flüssigkeitsdichte, jedoch in einer Richtung luftdurchlässige Anordnung zum Entfernen von angesaugter Luft aus dem Beutel aufweisen. Somit kann ein effektiveres Füllen des Beutels bewirkt werden. Der mit Blut gefüllte Beutel kann dann direkt für eine Blut-Retransfusion verwendet werden.

Im folgenden wird in Bezug auf die beigefügten Zeichnungen die Erfindung anhand von Ausführungsbeispielen näher beschrieben. Es zeigen:
- Fig. 1: eine Absaugvorrichtung gemäß der vorliegenden Erfindung mit verschlossenem Behälter, in dem der Beutel und der Schlauch mit dem Dichtungselement eingesteckt sind;
- Fig. 2: einen vergrößerten Ausschnitt mit der Deckel-Dichtung und dem in die Seitenwand des Behälters eingesteckten Dichtungselement,
- Fig. 3: eine Detaillansicht des Dichtungselements;
- Fig. 4: ein weiteres Ausführungsbeispiel für einen Beutel, wie er in der erfindungsgemäßen Absaugvorrichtung verwendet werden kann, und
- Fig. 5: eine Detaillansicht der Deckelkonstruktion des Behälters.

Fig. 1 zeigt einen Behälter 1, der z.B. aus Glas oder vorteilhaft durchsichtigem Kunststoff (z. B. Acrylglas) bestehen kann. Dieser Behälter 1 ist durch einen Deckel 2 vakuumdicht verschließbar. In den Behälter 1 ist ein Beutel 3 eingesetzt, an dem ein Schlauch 4 aus z. B. PVC, Silikon oder dgl. sterilisierbarem Material befestigt ist. Der Beutel 3 dient zur Aufnahme des durch den Schlauch 4 aus einer Wunde oder dgl. abgesaugten Wundsekretes, wie z.B. Blut, Eiter oder ähnliches. Beutel 3 und Schlauch 4 sind vorzugsweise einstückig ausgebildet. Der Beutel 3 und der Schlauch 4 bestehen weiterhin aus luftundurchlässigem Material. Der Schlauch 4 wird im eingesetzten Zustand durch eine Öffnung 5 mittels eines Dichtungselementes 6 aus dem Inneren des Behälters 1 nach außen geführt. Dabei ist das Dichtungselement 6 am Schlauch 4 befestigt, z.B. durch Klebstoff oder ähnliches. Das Dichtungselement 6 verschließt die Öffnung 5 zumindest bei geschlossenem Deckel 2 vakuumdicht. Die Öffnung 5 kann sich, wie dargestellt und vorzugsweise, im oberen Teil der Behälterwand, aber im Sinne einer kinematischen Umkehr auch im Deckel 2, befinden. An eine weitere Öffnung 7 in der Behälterwand oder im Deckel 2 kann eine Vakuumpumpe zum Abpumpen der Luft aus dem Inneren des Behälters 1 über einen Schlauch oder dgl. angeschlossen werden. Dies ist nur schematisch dargestellt. Diese Öffnung 7 ist mit einem Ventil 8 ebenfalls vakuumdicht verschließbar. Durch die vakuumdichte Ausgestaltung der Anordnung aus Dichtungselement 6, Behälter 1, Deckel 2 und Ventil 8 wird also ein einmal durch Abpumpen der Luft aus dem Inneren des Behälters 1 erzeugtes Vakuum bei geschlossenem Deckel 2 und bei geschlossenem Ventil 8 sehr lange aufrechterhalten, zumindest solange, bis der Beutel 3 routinemäßig entweder abhängig vom Füllungsgrad des Beutels 3 oder nach einigen Stunden entfernt wird. Das Vakuum im Inneren des Behälters 1 hat zur Folge, daß der vor dem Erzeugen des Vakuums in den Behälter 1 eingebrachte Beutel 3 gewissermaßen aufgebläht wird und am in die Wunde oder in das abzusaugende Sekret hineinragenden sondenartig ausgebildeten Ende des Schlauches 4 eine Saugwirkung erzeugt wird.

Das Dichtungselement 6 ist dabei in die Öffnung 5 eingesteckt. Ist der Beutel 3 mit Sekret gefüllt, kann er durch Öffnen des Deckels 2 (ggfs. nach Öffnen des Ventils 8) und durch einfaches Herausziehen der Dichtung 6 zusammen mit dem Schlauch 4 aus der Öffnung 5 leicht und einfach ausgewechselt werden. Der Beutel 3 mit Schlauch 4 und Dichtungselement 6 wird entsorgt. Eine Kontamination des Behälters 1 hat nicht stattgefunden.

Fig. 2 zeigt im Schnitt einen vergrößerten Ausschnitt von Fig. 1 mit dem in die Öffnung 5 eingesteckten Dichtungselement 6 und die Verbindung zwischen Behälterwand und Deckel 2 im Detail. Das Dichtungselement 6 ist im wesentlichen ringförmig mit jeweils einer (umlaufenden) Lippe 9, 10 an der Vorder- und der Rückseite. Diese beiden Lippen 9 und 10 erstrecken sich auf dem gesamten Umfang des Ringes radial nach außen. Im eingesteckten Zustand des Dichtungselementes 6 ist der die Öffnung 5 definierende Rand der Behälterwand (oder Deckelwand) zwischen diesen beiden Lippen eingeklemmt, wie in Fig. 2 zu sehen ist. Die Funktion der beiden Lippen ist also einerseits, das Dichtungselement 6 in der Öffnung 5 zu haltern, und andererseits, diese vakuumdicht zu verschließen. In einer bevorzugten Ausführungsform kann das Dichtungselement 6 zumindest auf einer Seite eine rohrförmige Verlängerung 11 aufweisen, die im eingesteckten Zustand des Dichtungselementes 6 ins Behälterinnere ragt. Dadurch wird ein Abknicken oder Beschädigen des durch das Dichtungselement 6 hindurch aus dem Behälterinneren nach außen geführten Schlauches 4 oder ein Lösen der festen Verbindung zwischen Schlauch 4 und Dichtungselement 6 verhindert.

Der Behälter 1 wird durch den Deckel 2 vakuumdicht verschlossen, indem der obere Rand des Behälters 1 z.B. in eine am Rand des Deckels 2 umlaufende Vertiefung oder Rille 17 eingreift. Dabei kann zusätzlich noch ein zwischen dem oberen Rand des Behälters 1 und dem Deckel 2 angeordneter Dichtungsring 16 für eine zuverlässige Abdichtung sorgen.

Wie erwähnt, ist die feste Verbindung zwischen dem Schlauch 4 und dem Dichtungselement 6 bzw. dessen rohrförmiger Verlängerung 11 von Bedeutung. Zwar ist es möglich, das Dichtungselement 6 mit rohrförmiger Verlängerung 11 gewissermaßen als Verstärkung des Schlauches 4 einteilig mit diesem zu fertigen. Jedoch ist dies aus technischen und Kostengründen äußerst problematisch. Zweckmäßig ist es daher, in der Medizintechnik übliche Materialien für den Schlauch 4 zu verwenden und das Dichtungselement 6 mit Verlängerung 11 aus einem thermoplastischen Kunststoff wie einen SBS-Kunststoff auf Styrolbasis herzustellen (beispielsweise aus der unter der Handelsbezeichnung Kraton der Firma Shell erhältlichen Formmasse) und mit dem Schlauch 4 einstückig zu verbinden. Dies kann durch eine Verklebung mittels eines Klebstoffes erfolgen oder durch Anlösen der miteinander zu verbindenden Flächen von Schlauch 4 und Dichtungselement 6 bzw. rohrförmiger Verlängerung 11. Falls das Dichtungselement aus einem Naturgummi wie Latex besteht, kann es zwar nicht mit dem Schlauch 4 verklebt werden, die dichte und feste Verbindung erfolgt dann über eine elastische Vorspannung, die von Dichtungselement 6 und Verlängerung 11 auf den Schlauch 4 ausgeübt wird.

Entscheidend für die Materialwahl, auch des Klebstoffs, ist, daß die Materialien einerseits ständig vakuumdicht sind und andererseits die Materialien auch bei der üblichen Sterilisierung nicht beeinflußt, insbesondere nicht zerstört werden.

Wie weiterhin in Fig. 1 dargestellt, ist es zweckmäßig, die Absaugvorrichtung mit zusätzlichen Elementen zu versehen, die vorzugsweise im Deckel 2 angeordnet sind, nämlich zum einen ein Anzeigeelement, das zum einen den Aufbau des Vakuums innerhalb des Behälters 1 anzeigt und zum anderen jedoch auch anzeigt, wenn das sich allmählich vollziehende Abbauen des Vakuums ein Ausmaß erreicht hat, das die Saugwirkung nicht mehr gewährleistet. Hier ist zweckmäßig, eine mittels einer federbelasteten Membran 12 gesteuerte mindestens zweifarbig gestaltete Kappe 13 im Deckel 2 anzuordnen. Die dicht eingeschweißte oder eingeklemmte Membran 12 nimmt bei hohem Vakuum eine unten liegende Stellung ein, in der auch die Kappe 13 nach unten gezogen ist. Mit abnehmendem Vakuum wird die Membran 12 nach außen gedrückt und drückt dadurch auch die Kappe 13 nach oben, so daß eine Sichtanzeige möglich ist. Ferner ist zweckmäßig, entweder das Ventil 8 selbst so ausgebildet, daß es zum Abbau des Vakuums innerhalb des Behälters 1 schnell zu öffnen ist, oder ein schnell zu öffnendes Lufteinlaßventil 14 zusätzlich vorzusehen, wie einen federbelasteten Verschlußstopfen, der durch Hebelbewegung aus seinem Sitz anhebbar ist.

Ferner ist es zweckmäßig, den Deckel 2 über ein Scharnier 15 an den Behälter 1 fest aber schwenkbar anzubringen, und ist zweckmäßig ein hakenförmiges oder laschenförmiges Verriegelungselement 18 am Deckel vorgesehen, das über eine Nase 16 des Behälters greifen kann und dabei den Dichtungsring 16 in der umlaufenden Rille 17 belastet.

Vorteilhaft sind die Kontur der Öffnung 5 und das Dichtungselement 6 in Ansicht im wesentlichen U-förmig, um das Dichtungselement 6 mit den beiden Lippen 9 und 10 in einfacher Weise bei geöffnetem Deckel 2 von oben in die Öffnung 5 einführen zu können. Der die Öffnung 5 definierende Rand kann dabei im Schnitt gesehen abgerundet sein, um das Anliegen der Lippen 9 und 10 bei eingestecktem Dichtungselement 6 zu erleichtern.

Falls mehrere Wunden eines Patienten abgesaugt und/oder verschlossen werden sollen, können mehrere Drainage-Katheter mittels eines T-Stücks zu dem einen Schlauch 4 zusammengeführt werden. Wie in Fig. 1 angedeutet, erfolgt diese Zusammenführung vorteilhafterweise außerhalb der Durchführung 5 des Behälters 3, da so nur eine einzige Durchführung eine Versorgung mehrerer Wunden des Patienten ermöglicht.

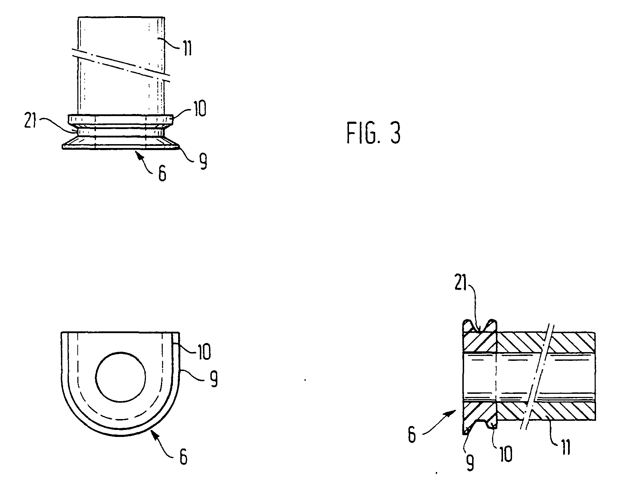

In Fig. 3 ist das Dichtungselement 6 in Auf-, Seiten- und Schnittansicht nochmals detailliert dargestellt. Die Vakuumdichtheit des Dichtungselements 6 ist ein wichtiger Faktor für den von einer Vakuumsauganlage unabhängigen Betrieb der erfindungsgemäßen Absaugvorrichtung. Wie in Fig. 3 dargestellt ist, weist das Dichtungselement 6 die rohrförmige Verlängerung 11 auf, die bei eingestecktem Zustand in das Behälterinnere ragt. Diese rohrförmige Verlängerung 11 verhindert ein Knicken des Schlauchs 4 im Bereich der Behälter-Öffnung 5. Darüber hinaus wird ein Verkanten oder Schiefliegen des Schlauchs 4 innerhalb des Dichtungselements 6 dadurch verhindert, daß die rohrförmige Verlängerung 11 bewirkt, daß der Schlauch 4 im Bereich des Dichtungselements 6 im wesentlichen immer senkrecht zu der Behälterwand liegt.

Das feste Verbinden des Schlauchs 4 mit sowohl Dichtungselement 6 als auch Verlängerung 11 verhindert ein Lösen dieser Verbindung durch Biegen oder Abknicken des Schlauchs 4 außerhalb des Behälters 1 bei in diesen eingesetzten Zustand.

Wie in Fig. 2 dargestellt ist, weist das Dichtungselement 6 auf der Seite, die beim Schließen des Deckels 2 von diesem angedrückt wird, eine Doppellippe 20 auf. Diese Doppellippe 20 wird vorteilhafterweise bei der Schließbewegung von einem Dichtring 16 des Deckels 2 angedrückt. Bei der in Fig. 3 dargestellten Ausführungsform weist das Dichtungselement 6 eine Doppellippe 9, 10 nur in dem Bereich auf, in dem das Dichtungselement 6 in den die Öffnung 5 festlegenden Rand eingreift. Wie in der Schnittansicht angedeutet kann die Doppellippe indessen auch vollständig umlaufend um das Dichtungselement 6 ausgebildet sein.

Der Umfang des Dichtungselements 6 im Bereich zwischen den beiden Lippen 9, 10 kann bei Betrachtung quer zu dem Dichtungselement 6 abgerundet oder im wesentlichen parallel zu der Achse des Dichtungselementes 6 sein. Dadurch wird ein besonders vorteilhaftes Zusammenwirken mit dem die Öffnung 5 definierenden Rand des Behälters erreicht. Für ein sicheres Einklemmen des Dichtungselementes 6 in dem die Öffnung 5 festlegenden Rand des Behälters 2 kann die in eingestecktem Zustand äußere Lippe 9 einen größen Durchmesser aufweisen als die innere Lippe 10.

Wenigstens eine der beiden Lippen 9, 10 kann bei Schnittansicht abgerundet sein oder eben auslaufen, um das bei einer spitz zulaufenden Lippe erhöhte Risiko einer Beschädigung der Lippe zu vermeiden.

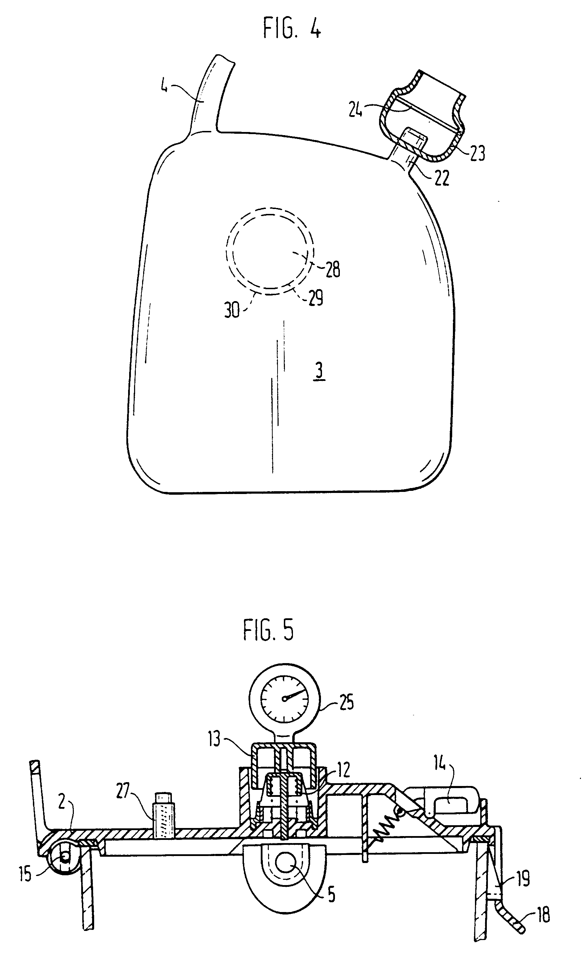

Fig. 4 zeigt eine Ausführungsform des Beutels 3, wie er insbesondere für den intraoperativen Einsatz der Absaugvorrichtung vorteilhaft ist. Bei dieser Verwendung wird während einer Operation Blut aus wenigstens einer Wunde eines Patienten in den Beutel 4 des Behälters 3 abgesaugt. Der Beutel 3 weist dabei vorteilhafterweise ein Volumen von beispielsweise 500 ml auf, wie es für Bluttransfusionsbeutel üblich ist. Da bei dieser Anwendung der erfindungsgemäßen Absaugvorrichtung oft ein gewisser Anteil an Luft mit eingesaugt wird, ist es zum effizienten Füllen des Beutels 4 von Vorteil, wenn dieser eine Anordnung zum Entfernen von angesaugter Luft aus dem Beutel 4 in das Innere des Behälters 1 aufweist. Wie dargestellt, kann dies beispielsweise durch einen angesetzten Stutzen 22 erfolgen, an dem ein fester Träger 23 angebracht ist, der eine luftdurchlässige jedoch flüssigkeitsdichte Membran 24 spannt. Von großem Vorteil ist es, wenn diese Membran 24 Feuchtigkeit oder gar Keime von einem Verlassen des Behälters 3 durch den Öffnungsstutzen 22 abhält und die durch die Membran 24 tretende Luft somit eine gewisse Sterilisierung erfährt. Dies kann beispielsweise durch Membrane mit Mikroporen geschehen, wie sie beispielsweise unter dem Handelsnamen Gore-Tex kommerziell erhältlich sind. Alternativ kann eine solche Membran 28 einen Teil der Wand des Beutels 3 bilden. Die Membran 28 kann beispielsweise in einen Ausschnitt 29 des Beutels 3 randseitig eingeschweißt (30) sein.

Fig. 5 zeigt detailliert den Deckel 2 des Behälters 1 der erfindungsgemäßen Absaugvorrichtung. Bezugnehmend auf Fig. 1 wurde schon ausgeführt, daß mittels einer federbelasteten Membran 12 und einer beispielsweise zweifarbig gestalteten Kappe 13 eine Sichtanzeige für das Vakuum in dem Behälter 1 ausgebildet sein kann. Für eine genaue Kontrolle des Vakuums in dem Behälter 1 kann zur Anzeige des Vakuums wie in Fig. 5 dargestellt, auch eine digitale oder analoge Anzeigeeinrichtung 25 des Vakuums vorgesehen sein. Es kann auch die Kappe 13 mit einer entsprechenden Skala versehen sein (nicht dargestellt).

Wie in Fig. 5 weiterhin dargestellt, kann im Deckel 2 oder in der Wand des Behälters 1 eine handbetätigbare Ventileinrichtung 27 vorgesehen sein, die es gestattet, einen minimalen Innendruck für den Behälter 2 einzustellen. Bei einer automatisch arbeitenden Ventileinrichtung 27 im Sinne eines Sicherheitsventils (Überdruckventil) kann ein bestimmter Grenzwert des Innendrucks festgelegt werden. Allerdings kann alternativ auch das Lufteinlaßventil 14 in geeigneter Weise bemessen und ausgebildet sein, obgleich es beim Ausführungsbeispiel zur Schnellentlastung dient. Somit kann der Behälter 2 leicht und schnell an einer Pumpstation abgepumpt werden, ohne daß die Gefahr der Erzeugung eines zu niedrigen Innendrucks im Behälter 2 besteht, was eine Beeinträchtigung oder gar Beschädigung des Behälters 2 zur Folge hätte. Zweckmäßig, jdeoch nicht im einzelnen dargestellt, sind Sicherungsmittel vorgesehen, die eine Manipulation insbesondere der die Abdichtung erzielenden Elemente durch Unbefugte verhindern.

Zusammenfassend wird also eine Ansaugvorrichtung mit einsetzbarem Einweg-Beutel mit angeschlossenem Einweg-Schlauch angegeben, bei der im Betrieb es lediglich erforderlich ist, einmal ein Vakuum aufzubauen, ohne daß es erforderlich wäre, eine Vakuumpumpe ständig angeschlossen halten zu müssen. Die erfindungsgemäße Ansaugvorrichtung eignet sich daher insbesondere für ambulanten Gebrauch und kann auch von nicht bettlägrigen Patienten verwendet und von diesen mitgeführt werden. Entscheidend ist dabei, daß das Vakuum solange aufrechterhalten werden kann, bis üblicherweise, d. h. routinemäßig, der Beutel ausgetauscht wird.

## Patentansprüche

1. Ambulant verwendbare Sekret-Absaugvorrichtung, mit
einem durch einen Deckel (2) vakuumdicht verschließbaren Behälter (1),
einem in den Behälter (1) einsetzbaren, einen Schlauch (4) aufweisenden, flüssigkeits- und vakuumdichten Beutel (3),
einer ersten Öffnung (7) in der Wand des Behälters (1), über die in dem verschlossenen Behälter (1) ein Vakuum erzeugbar ist und die mittels eines Ventils (8) vakuumdicht verschließbar ist, wodurch der verschlossene Behälter (1) von einer Vakuumquelle nach Erzeugung des Vakuums abtrennbar ist,
und
einer Durchführung nach außerhalb der Vorrichtung für den Schlauch (4) zum Saugen von Sekret oder dergleichen in den Beutel (3) mittels des in dem verschlossenen Behälter (1) erzeugten Vakuums,
**dadurch gekennzeichnet,**
**daß** die Durchführung gebildet ist durch
- eine zweite Öffnung (5) im oberen Teil der Wand von Behälter (1) oder Deckel (2), und
- ein mit dem Schlauch fest verbundenes, unabhängiges Dichtungselement (6), das bei geöffnetem Behälter (1) in die zweite Öffnung (5) so einsteckbar ist, daß bei der Schließbewegung des Deckels (2) gegenüber dem Behälter (1) diese am Ende der Schließbewegung im wesentlichen in einer Richtung senkrecht zu einer Längsachse des Dichtungselements (6) mit diesem in Anlage bringbar sind, und das eine in eingestecktem Zustand in das Innere des Behälters (1) ragende rohrförmige Verlängerung (11) aufweist.

2. Absaugvorrichtung nach Anspruch 1,
**dadurch gekennzeichnet,**
**daß** der Deckel (2) einen Dichtring (16) aufweist, der bei geschlossenem Deckel (2) gegen das Dichtungselement (6) gedrückt ist.

3. Absaugvorrichtung gemäß einem der vorhergehenden Ansprüche,
**dadurch gekennzeichnet,**
**daß** der Schlauch (4) mit dem Dichtungselement (6) verklebt oder verschweißt ist.

4. Absaugvorrichtung gemäß einem der vorhergehenden Ansprüche,
**dadurch gekennzeichnet,**
**daß** der Schlauch aus Polyvinylchlorid oder dgl. sterilisierbarem Material besteht.

5. Absaugvorrichtung gemäß einem der Ansprüche 1 bis 4,
**dadurch gekennzeichnet,**
**daß** das Dichtungselement (6) aus einem thermoplastischen SBS-Kunststoff oder dgl. sterilisierbarem Material besteht.

6. Absaugvorrichtung nach einem der Ansprüche 1 bis 4,
**dadurch gekennzeichnet,**
**daß** das Dichtungselement (6) aus einem Naturgummi wie Latex gefertigt ist.

7. Absaugvorrichtung nach einem der vorhergehenden Ansprüche,
**dadurch gekennzeichnet,**
**daß** das Dichtungselement (6) an der in Richtung des Deckeis (2) weisenden Seite eine Doppellippe (20) aufweist, die bei der Schlleßbewegung des Deckels (2) durch diesen vakuumdicht andrückbar ist.

8. Absaugvorrichtung nach einem der vorhergehenden Ansprüche,
**dadurch gekennzeichnet,**
**daß** das Dichtungselement (6) eine Doppellippe (9,10) aufweist, in die in eingestecktem Zustand der die Öffnung (5) festlegende Rand einklemmbar ist.

9. Absaugvorrichtung nach einem der Ansprüche 1 bis 8,
**dadurch gekennzeichnet,**
**daß** das Dichtungselement (6) eine umlaufende Doppellippe (9,10) aufweist.

10. Absaugvorrichtung nach einem der Ansprüche 7 bis 9,
**dadurch gekennzeichnet,**
**daß** der Umfang des Dichtungselements (6) im Bereich zwischen den beiden Lippen (9,10;20) bei Betrachtung quer zu dem Dichtungselement (6) abgerundet ist oder im wesentlichen parallel zu der Achse des Dichtungselements (6) ist.

11. Absaugvorrichtung nach einem der Ansprüche 1 bis 10,
**dadurch gekennzeichnet,**
**daß** eine die Doppellippe und die rohrförmige Verlängerung (11) einstückig mit dem Dichtungselement (6) ausgebildet sind.

12. Absaugvorrichtung nach einem der Ansprüche 8 bis 11,
**dadurch gekennzeichnet,**
**daß** die in eingesetztem Zustand äußere Lippe (9) des Dichtungselements (6) einen größeren Durchmesser aufweist als die innere Lippe.

13. Absaugvorrichtung nach einem der vorhergehenden Ansprüche,
**dadurch gekennzeichnet,**
**daß** sie eine Anzeigeeinrichtung zur Anzeige des Innendrucks des Behälters (1) aufweist.

14. Absaugvorrichtung nach einem der vorhergehenden Ansprüche,
**dadurch gekennzeichnet,**
**daß** sie eine Einrichtung zur Anzeige des Unter- und /oder überschreitens eines vorbestimmten Wert des Innendrucks in dem Behälter (2) aufweist.

15. Absaugvorrichtung nach einem der vorhergehenden Ansprüche,
**dadurch gekennzeichnet,**
**daß** sie eine Vorrichtung zur Einstellung des minimalen Innendrucks des Behälters (1) aufweist.

16. Absaugvorrichtung nach einem der vorhergehenden Ansprüche,
**dadurch gekennzeichnet,**
**daß** wenigstens zwei absaugseitige Schläuche mittels einem vakuumdichten T-Stück zu dem einen Schlauch (4) zusammengeführt sind.

17. Absaugvorrichtung nach Anspruch 16,
**dadurch gekennzeichnet,**
**daß** die wenigstens zwei absaugseitigen Schläuche außerhalb des Behälters (1) zu dem einen Schlauch (4) zusammengeführt sind.

18. Absaugvorrichtung nach einem der vorhergehenden Ansprüche,
**dadurch gekennzeichnet,**
**daß** der Beutel (3) den Schlauch (4) als einzige Öffnung aufweist und im übrigen gegenüber dem Inneren des Behälters (1) vakuumdicht ist.

19. Absaugvorrichtung nach einem der Ansprüche 1 bis 17,
**dadurch gekennzeichnet,**
**daß** der Beutel (3) ferner eine flüssigkeitsdichte jedoch in eine Richtung luftdurchlässige Anordnung (22, 23, 24; 28, 29, 30) zum Entfernen von angesaugter Luft aus dem Beutel (3) in das Innere des Behälters (1) aufweist.

20. Absaugvorrichtung nach Anspruch 19,
**dadurch gekennzeichnet, daß** der Beutel (3) eine gehalterte Membran (24; 28) aufweist.

21. Absaugvorrichtung nach Anspruch 20,
**dadurch gekennzeichnet,**
**daß** die Membran (24) in eine steife Halterung (23) eingesetzt ist, die an einem in den Beutel (3) führenden Schlauchstutzen (22) befestigt ist.

22. Absaugvorrichtung nach Anspruch 20,
**dadurch gekennzeichnet,**
**daß** die Membran (28) in eine Ausnehmung (29) des Beutels eingeschweißt (30) ist.

## Claims

1. Secretion suction draw off device for outpatient use having
a container (1) vacuumtightly closable by means of a lid (2),
a liquidtight and vacuum-tight bag (3) with a tube (4), which can be placed in the container (1),
a first opening (7) in the wall of said container (1), through which a vacuum can be generated in the container (1) when closed and which can be closed in a vacuum-tight manner by means of a valve (8) whereby the closed container (1) can be separated from the source of vacuum after generation of the vacuum, and
a passage to the outside of the container for said tube (4) for drawing the secretion or the like into the bag (3) by means of the vacuum generated is said closed container (1),
**characterised in that** said passage if formed by
- a second opening (5) in the upper part of the wall of the container (1) or the lid (2), and
- a separate sealing element (6) fastened to the tube, which can be inserted into said second opening (5) such that upon closure of the lid (2) in relation to the container (1) those can be brought - at end of the closing movement substantially in a direction perpendicular to the longitudinal axis of the sealing element (6) - into abutment with it and which comprises a pipe-like extension (11) which in the inserted condition projects into the container (1) interior.

2. Suction draw off device according to claim 1, **characterised in that**,
the lid (2) has a sealing ring (16) which is pressed against the sealing element (6) when the lid (2) is closed.

3. Suction draw off device according to any preceding claim,
**characterised in that**,
the tube (4) is glued or welded to the sealing element (6).

4. Suction draw off device according to any preceding claim,
**characterized in that**,
the tube is of polyvinylchloride or like sterilizable material.

5. Suction draw off device according to any of claims 1 to 4,
**characterized in that**,
the sealing element (6) is of a thermoplastic SBS plastics or like sterilizable material.

6. Suction draw off device according to any of claims 1 to 4,
**characterized in that**,
the sealing element (6) is manufactured of a natural rubber such a latex.

7. Suction draw off device according to any preceding claim,
**characterized in that**,
the sealing element (6) has, at the side directed towards the lid (2), a double lip (20) that upon the closure movement of the lid (2) can be pressed on by the lid a vacuum-tight manner.

8. Suction draw off device according to any preceding claim,
**characterized in that**,
the sealing element (6) has a double lip (9, 10) into which, in the inserted condition, the edge defining the opening (5) can be clamped.

9. Suction draw off device according to any of claims 1 to 8,
**characterized in that**,
the sealing element (6) has a circumferential double lip (9, 10).

10. Suction draw off device according to any of claims 7 to 9,
**characterized in that**,
that the circumference of the sealing element (6), in the region between the two lips (9, 10; 20), viewed transversely to the sealing element (6), is rounded or is in substance parallel to the axis of the sealing element (6).

11. Suction draw off device according to any of claims 1 to 10,
**characterized in that**,
the double lip and the pipe-like extension (11) are formed in one piece with the sealing element (6).

12. Suction draw off device according to any of claims 8 to 11,
**characterized in that**,
the lip (9) of the sealing element (6) which is outermost in the inserted condition has a greater diameter than the inner lip.

13. Suction draw off device according to any preceding claim,
**characterized in that**,
it has an indicator device for indicating the internal pressure of the container (1).

14. Suction draw off device according to any preceding claim,
**characterized in that**,
it has a device for indicating that a predetermined value of the internal pressure in the container (2) has been undershot and/or exceeded.

15. Suction draw off device according to any preceding claim,
**characterized in that**,
it has a device for setting the minimum internal pressure of the container (1).

16. Suction draw off device according to any preceding claim,
**characterized in that**,
at least two tubes on the suction draw off side are brought together by means of a vacuum-tight T-piece to said one tube (4).

17. Suction draw off device according to claim 16,
**characterized in that**,
the at least two suction draw off side tubes are brought together to said one tube (4) outside of the container (1).

18. Suction draw off device according to any preceding claim,
**characterized in that**,
the bag (3) has the tube (4) as sole opening, and is otherwise sealed in a vacuum-tight manner with regard to the interior of the container (1).

19. Suction draw off device according to any of claim 1 to 17,
**characterized in that**,
the bag (3) further has an arrangement (22, 23, 24; 28, 29, 30) for the removal of drawn-in air from the bag (3) into the interior of the container (1), which arrangement is liquid-tight but air permeable in one direction.

20. Suction draw off device according to claim 19,
**characterized in that**,
thebag (3) comprises a mounted membrane (24; 28).

21. Suction draw off device according to claim 20,
**characterized in that**,
the membrane (24) is placed in a stiff mount (23) which is attached to a tube connection (22) leading into the bag (3).

22. Suction draw off device according to claim 20,
**characterized in that**,
the membrane (28) is welded (30) into a cut-out (29) of the bag.

## Revendications

1. Dispositif d'aspiration de sécrétions utilisable en ambulatoire, comportant
un récipient (1) verrouillable de façon étanche au vide par un couvercle (2),
une poche (3) étanche aux liquides et au vide, utilisable dans le récipient (1), présentant un tube (4),
un premier orifice (7) dans la paroi du récipient (1) par lequel il est possible de créer un vide dans le récipient (1) fermé et qui est verrouillable de façon étanche au vide au moyen d'une soupape (8), le récipient fermé (1) étant de ce fait séparable d'une source de vide après création du vide, et
un passage vers l'extérieur du dispositif pour le tube (4) en vue de l'aspiration de sécrétions ou similaires dans la poche (3) au moyen du vide créé dans le récipient (1) fermé,
**caractérisé en ce que** le passage est formé par
- un second orifice (5) dans la partie supérieure de la paroi du récipient (1) ou du couvercle (2),
et
- un élément d'étanchéité (6) indépendant, solidaire du tube, qui, lorsque le récipient (1) est ouvert, peut être inséré dans le deuxième orifice (5) de façon que, lors du mouvement de fermeture du couvercle (2) par rapport au récipient (1), ceux-ci peuvent, à la fin du mouvement de fermeture sensiblement dans une direction perpendiculaire à l'axe longitudinal de l'élément d'étanchéité (6), venir en appui avec ce dernier, et qui présente à l'état inséré un prolongement (1) tubulaire dépassant à l'intérieur du récipient (1).

2. Dispositif d'aspiration selon la revendication 1, **caractérisé en ce que** le couvercle (2) présente un joint (16) qui est comprimé contre l'élément d'étanchéité (6) lorsque le couvercle (2) est fermé.

3. Dispositif d'aspiration selon une des revendications précédentes, **caractérisé en ce que** le tube (4) est collé ou soudé à l'élément d'étanchéité (6).

4. Dispositif d'aspiration selon une des revendications précédentes, **caractérisé en ce que** le tube est en chlorure de polyvinyle ou autre matériau stérilisable similaire.

5. Dispositif d'aspiration selon une des revendications 1 à 4, **caractérisé en ce que** l'élément d'étanchéité (6) est en une matière synthétique SBS thermoplastique ou autre matériau stérilisable similaire.

6. Dispositif d'aspiration selon une des revendications 1 à 4, **caractérisé en ce que** l'élément d'étanchéité (6) est en caoutchouc naturel tel que le latex.

7. Dispositif d'aspiration selon une des revendications précédentes, **caractérisé en ce que** l'élément d'étanchéité (6) présente sur le côté orienté dans la direction du couvercle (2) une double lèvre (20) qui, lors du mouvement de fermeture du couvercle (2), peut être comprimée de façon étanche au vide par ce dernier.

8. Dispositif d'aspiration selon une des revendications précédentes, **caractérisé en ce que** l'élément d'étanchéité (6) présente une double lèvre (9, 10) dans laquelle, à l'état inséré, le bord définissant l'orifice (5) peut être pincé.

9. Dispositif d'aspiration selon une des revendications 1 à 8, **caractérisé en ce que** l'élément d'étanchéité (6) présente une double lèvre (9, 10) périphérique.

10. Dispositif d'aspiration selon une des revendications 7 à 9, **caractérisé en ce que** la circonférence de l'élément d'étanchéité (6) est arrondie dans la zone entre les deux lèvres (9, 10 ; 20), considérée perpendiculairement à l'élément d'étanchéité (6), ou est sensiblement parallèle à l'axe de l'élément d'étanchéité (6).

11. Dispositif d'aspiration selon une des revendications 1 à 10, **caractérisé en ce qu'**une/la double lèvre et le prolongement tubulaire (11) sont formés d'une seule pièce avec l'élément d'étanchéité (6).

12. Dispositif d'aspiration selon une des revendications 7 à 11, **caractérisé en ce que** la lèvre extérieure (9) de l'élément d'étanchéité (6) à l'état inséré présente un diamètre supérieur à celui de la lèvre intérieure.

13. Dispositif d'aspiration selon une des revendications précédentes, **caractérisé en ce qu'**il présente un dispositif d'affichage pour afficher la pression interne du récipient (1).

14. Dispositif d'aspiration selon une des revendications précédentes, **caractérisé en ce qu'**il présente un dispositif pour l'affichage de la non-atteinte et/ou du dépassement d'une valeur prédéterminée de la pression interne dans le récipient (1).

15. Dispositif d'aspiration selon l'une des revendications précédentes, **caractérisé en ce qu'**il présente un dispositif pour régler la pression interne minimale du récipient (1).

16. Dispositif d'aspiration selon une des revendications précédentes, **caractérisé en ce qu'**au moins deux tubes du côté aspiration sont réunis en un tube (4) au moyen d'une pièce en T étanche au vide.

17. Dispositif d'aspiration selon la revendication 16, **caractérisé en ce que** les au moins deux tubes du côté aspiration sont réunis en un tube (4) à l'extérieur du récipient (1).

18. Dispositif d'aspiration selon une des revendications précédentes, **caractérisé en ce que** la poche (3) présente le tube (4) comme seul orifice et est par ailleurs étanche au vide par rapport à l'intérieur du récipient (1).

19. Dispositif d'aspiration selon une des revendications 1 à 17, **caractérisé en ce que** la poche (3) présente en outre un dispositif (22, 23, 24 ; 28, 29, 30) étanche aux liquides, mais perméable à l'air dans une direction, en vue de l'évacuation de l'air aspiré hors de la poche (3) dans le récipient (1).

20. Dispositif d'aspiration selon la revendication 19, **caractérisé en ce que** la poche (3) présente une membrane (24 ; 28) maintenue.

21. Dispositif d'aspiration selon la revendication 20, **caractérisé en ce que** la membrane (24) est placée dans une retenue (23) rigide fixée à une tubulure (22) menant à l'intérieur de la poche (3).

22. Dispositif d'aspiration selon la revendication 20, **caractérisé en ce que** la membrane (28) est soudée dans un évidement (29) de la poche.
